# EUROPEAN PATENT APPLICATION

(11) **EP 4 674 379 A1**
(43) Date of publication of application: **07.01.2026**
(21) Application number: 24763229.2
(22) Date of filing: 29.02.2024
(51) Int. Cl.: A61F 2/07

(54) **BRANCH ARTERY RECONSTRUCTION ASSISTING DEVICE AND S-C BRANCH-FIRST AORTA RECONSTRUCTION SYSTEM**

(30) Priority: 01.03.2023 CN 202310201683
(71) Applicant: Fuwai Hospital, CAMS & PUMC, Beijing 100037 (CN); APT Medical Inc., Xiangtan, Hunan 411499 (CN)
(72) Inventor: SHU, Chang, Beijing 100037 (CN); QIU, Yuchen, Xiangtan, Hunan 411499 (CN)
(74) Representative: Dehns Germany Partnerschaft mbB
(86) International application number: PCT/CN2024/079393
(87) International publication number: WO 2024/179549

(57) **Abstract**

The present disclosure provides a branch artery reconstruction assist device and an S-C branch-first aortic reconstruction system. The branch artery reconstruction assist device comprises a first stent body; the first stent body comprises a bare stent segment, and a proximal-side stent graft segment and a distal-side stent graft segment connected to two ends of the bare stent segment; wherein each of the proximal-side stent graft segment and the distal-side stent graft segment is provided with an occlusion membrane and at least one first stent graft channel; the occlusion membrane comprises a first opening, and the first opening is correspondingly connected to a first end of the first stent graft channel, and a second end of the first stent graft channel is connected to a corresponding branch artery stent graft to form a branch channel so as to pass through the bare stent. According to the present disclosure, the second stent body and the first stent body achieve sealing cooperation to isolate lesions, thereby ensuring that endoleak does not occur in the stents and ensuring the patency of various arteries, and normal supply of cerebral blood flow and/or organ blood flow during surgery, thereby avoiding vascular exclusion, and further reducing complications caused by ischemia.

## Description

### Technical Field

The present disclosure relates to the technical field of medical instruments, and in particular to a branch artery reconstruction assist device and an S-C branch-first aortic reconstruction system.

### Background

Aorta is the thickest arterial duct in the human body, is a main access for conveying blood to various parts of the whole body, starts from the left ventricle of the heart, is slightly arched upwards, rightwards and then downwards, then runs downwards along the spine, and is branched to obtain many smaller arteries in the thoracic lumen and the abdominal lumen. That is, the aorta mainly comprises several parts, i.e. ascending aorta, aortic arch, and thoracoabdominal aorta (the thoracoabdominal aorta refers to the aorta after and below the left subclavian artery and above the iliac arteries at two sides, the aorta above the diaphragm is referred to as the descending thoracic aorta, and the aorta below the diaphragm is referred to as the abdominal aorta). Dangerous aortic lesions mainly comprise aortic aneurysms and aortic dissections. In clinical practice, aortic arch lesions (mainly aortic aneurysms or aortic dissections) have always been a major problem for the treatment of vascular diseases, and have extremely high non-operative mortality. However, there are many problems in current treatment means, and death, paraplegia, cerebral embolism and acute renal failure all happen occasionally. If a surgical treatment method is used, there are many anastomotic stomas, the degree of difficulty of anastomosis is large, hemorrhage during operation and deep hypothermia circulatory arrest occur, and a series of serious complications occur.

With the rapid development of thoracic endovascular aortic repair (TEVAR) technology and devices, the indications of TEVAR are expanded from descending aortic lesions to aortic arch lesions. However, aortic arch diseases are always a forbidden zone for endovascular interventional treatment due to its complex anatomy, and the main difficulty thereof lies in how to ensure isolation of aortic arch lesions while maintaining vascular patency of branches on the arch. Interventional treatment has many off-label instrument applications, and currently, multiple endovascular aortic reconstruction techniques have been tried, comprising fenestrated stents, customized stents, chimney techniques and "skirt stents" specifically designed for the chimney technique, and endovascular reconstruction of modular branch artery stent grafts, etc. However, the problem of intraoperative blood exclusion, the long-term patency rate of the branch artery stent graft, and the like are not satisfactory, and currently there is no mature endovascular treatment product which can be widely applied to the clinic.

Currently, in clinical practice, endovascular treatment is performed on aortic dissections or aneurysms involving an aortic arch segment mainly by the assistance of the "chimney" technique and "fenestration" technique, but a common difficulty comprises "endoleak" between a main body and a branch artery stent graft and mismatch therebetween, which brings a great challenge to clinical treatment, and still does not solve potential risks such as endoleak and stent displacement.

In order to solve the problems of endoleak in the chimney technology and the long-term patency rate of the branch artery stent graft, on the basis of deep insights into clinical requirements, the inventor has developed and researched that in an aortic arch stent system (fenestration type) and a CSkirt aortic arch branch artery stent graft system (fenestration-type skirt stent), an aortic arch main body stent is punctured through an aortic stent graft membrane-breaking system to form a puncture point, a balloon is then used to expand the puncture point to form a "window" through which blood can flow, and then the CSkirt aortic arch branch artery stent graft (fenestration-type skirt stent) is fed along a guidewire to pass through the "window" and is released. During use, it has been found that such a structure has problems such as complicated operation.

On the other hand, aortic diseases involving visceral artery branches (the visceral artery branches mainly refer to a range of the renal artery, celiac artery and superior mesenteric artery) mainly comprise diseases such as Thoracoabdominal Aortic Aneurysm (TAAA), Aortic dissection (AD) and Abdominal Aortic Aneurysm (AAA), etc. A common feature of lesions, such as thoracoabdominal aortic aneurysms and some aortic dissections and abdominal aortic aneurysms, is that these lesions involve visceral branch arteries, or may involve the region of these visceral artery branches in the treatment process.

Taking the thoracoabdominal aortic aneurysm as an example, the thoracoabdominal aortic aneurysm refers to an aneurysm-like dilated disease involving both a thoracic aorta and an abdominal aorta, and the lesion region thereof is complex and extensive, and generally involves important visceral vessels such as an intercostal artery, a celiac trunk, a superior mesenteric artery, or renal arteries on both sides.

Currently, conventional abdominal endovascular aneurysm repair (EVAR) still has difficulties in the treatment of perirenal abdominal aortic aneurysm, of thoracoabdominal aortic aneurysms and aortic dissections involving abdominal aortic branch vessels, in which how to reconstruct visceral arterial blood supply is a major obstacle. For aortic diseases involving visceral artery branches, due to the perfusion and blood supply relation of visceral arteries, a simple stent graft cannot be used for treatment. The endovascular repair technique reconstructs blood supply for visceral artery branches, it reduces enormous trauma caused by open surgery, and plays an important role in treating aortic diseases involving visceral artery branches, and the endovascular repair techniques used thereby mainly comprise chimney, sandwich, fenestration and multi-branch techniques.

At the earliest, a chimney stent-graft is used as a remedial measure in cases where a stent-type artificial vessel covers a branch artery unexpectly. Currently, the chimney stent-graft is mainly applied to perirenal abdominal aortic aneurysm patients whose physical conditions are not suitable for open surgery, and whose anatomical conditions are not suitable for fenestration, and treatment by multi-branch technique. Since the chimney stent is located between the stent-type artificial vessel and an autologous arterial wall, the occurrence of endoleak is high; and although the sandwich technique shows a good recent effect, the long term stability and effectiveness still need to be further verified. The fenestration technology has strict requirements on the patient's aneurysm anatomical conditions, where an aneurysm neck with a too large twisting angle will affect accurate apposition of the window with a target vessel. Same as the fenestration technology, the multi-branch technology has complex operation and high cost, and needs to be customized individually, and is currently only applied to a few patients.

Currently, methods for processing TAAA mainly comprise open surgery and hybrid surgery. Since both open surgery and hybrid surgery cannot avoid laparotomy and cause enormous trauma, and the reconstruction of visceral arteries is complicated, currently, there are still risks of massive haemorrhage, shock, cardiac arrest and multiple organ failure during the perioperative period.

### Summary

In view of this, an object of some embodiments of the present disclosure is to provide a branch artery reconstruction assist device and a branch-first aortic reconstruction system, which are used for solving the problems of serious complications caused by endoleak, organ ischemia during operation, etc. which are still unavoidable in the related art.

On the basis of the object above, some embodiments of the present disclosure provide a branch artery reconstruction assist device, comprising a first stent body; the first stent body comprises a bare stent segment, and a proximal-side stent graft segment and a distal-side stent graft segment connected to two ends of the bare stent segment; wherein each of the proximal-side stent graft segment and the distal-side stent graft segment is provided with an leak proof membrane and at least one first stent graft channel; the occlusion membrane comprises a first opening, and the first opening is correspondingly connected to a first end of the first stent graft channel, and a second end of the first stent graft channel is connected to a corresponding branch artery stent graft to form a branch channel so as to pass through the bare stent. Here, the number of the first openings is the same as the number of the first stent graft channels. The plurality of first openings and the plurality of first stent graft channels are respectively provided in one-to-one correspondence, so as to respectively achieve the reconstruction of blood flow channels in corresponding branch arteries.

Here, the first stent graft channels may be formed by corresponding embedded stent grafts, or other structures that can be used to individually connect the aorta to branch arteries thereof in the related art. Each of the embedded stent grafts may comprise an embedded stent body having a lumen and a covering membrane provided outside the embedded stent body. That is, the first stent graft channel is formed by a lumen of a corresponding embedded stent graft. Here, the bare stent segment is a stent without a covering membrane.

Here, the cross section of the first stent graft channel is circular or elliptical, such that the shape of the first stent graft channel can match the shape of branch arteries or branch artery stent grafts, and there is no slit after attachment, no leakage of blood occurs.

When the number of the first stent graft channels provided in the proximal-side stent graft segment or the distal-side stent graft segment is multiple, the multiple first stent graft channels are arranged parallel to each other. Specifically, the number of the first stent graft channels in the proximal-side stent graft segment may be zero, one, two, three, and so on; and the number of the first stent graft channels in the distal-side stent graft segment may also be zero, one, two, three, and so on. The number of the first stent graft channels is determined according to the placement position of stent and the usage habits of doctors, and the total number of first stent grafts in the first stent body is also consistent with the number of branch artery accesses requiring to be reconstructed.

Preferably, the distance between an inlet end of a branch artery corresponding to the proximal-side stent graft segment and an inlet end of a branch artery corresponding to the distal-side stent graft segment is smaller than the length of the bare stent.

Preferably, the bare stent has a bendable structure.

Here, the branch arteries are provided with branch artery stent grafts, and some of blood flow channels formed by the branch artery stent grafts communicating with the first stent graft channels are located in the bare stent segment, and some are located outside the bare stent segment. An overlapping region nested with the proximal side of a corresponding branch artery stent graft is provided at any position of the first stent graft channel. In this way, a corresponding branch channel can be formed by nesting and overlapping the first stent graft channel with a corresponding branch artery stent graft, thereby improving the stability of a branch artery vessel access, and better completing the reconstruction of an artery branch vessel without blood leakage.

In the overlapping region, the branch artery stent graft and the corresponding first stent graft channel abut against each other, and there is no gap between the abutting surface, thereby preventing blood leakage and preventing the branch artery stent graft from falling off. The length of the overlapping region is greater than a certain preset size, so as to ensure that there is a sufficient contact surface between the first stent graft channel and the branch artery stent graft, thereby avoiding blood leakage, improving the ability of the branch artery stent graft to resist blood flow impact, and improving the stability of the branch artery stent graft. For example, the preset size may be any length that can be used to increase the stability of connection between the first stent graft channel and a corresponding branch artery stent graft. Of course, barbs fixed on the inner wall of the first stent graft channel may also be preset on the branch artery stent graft, so as to prevent the branch artery stent graft from falling off caused by blood flow impact.

Preferably, the connection position between the proximal-side stent graft segment or the distal-side stent graft segment and the occlusion membrane is flush with the first end of the first stent graft channel, the occlusion membrane is in a planar shape; and/or when the connection position between the proximal-side stent graft segment or the distal-side stent graft segment and the occlusion membrane is not flush with the first end of the first stent graft channel, the occlusion membrane is in an inclined surface shape.

That is to say, the end of the first stent graft channel away from the branch artery may be flush or not flush with the endpoint in the direction of the proximal-side stent graft segment or the distal-side stent graft segment away from the bare stent segment; while the occlusion membrane is perpendicular or not perpendicular relative to the covering membrane direction of the first or distal-side stent graft segment. It needs to be emphasized that the design of the inclined surface shape facilitates the blood flow to enter the first stent graft channel, but the design of the planar design will not prevent the blood flow from entering the first stent graft channel.

Preferably, the occlusion membrane in the proximal-side stent graft segment is connected to a proximal end of the proximal-side stent graft segment, and the occlusion membrane in the distal-side stent graft segment is connected to a distal end of the distal-side stent graft segment. Here, the occlusion membrane may be provided at the position of the proximal end of the proximal-side stent graft segment and the distal end of the distal-side stent graft segment.

Preferably, a first positioning stent is provided at the proximal end of the proximal-side stent graft segment and/or at the distal end of the distal-side stent graft segment. Specifically, the first positioning stent is generally arranged at a position on the proximal side of the first stent body, that is, when the proximal-side stent graft segment is at the proximal side, the first positioning stent is provided at a first end of the proximal-side stent graft segment; and when the distal-side stent graft segment is at the distal side, the first positioning stent is provided at a second end of the distal-side stent graft segment. When the first stent body is delivered, a stent delivery system is connected to the first positioning stent for deploying the first stent body to a target position.

Here, the end of the proximal-side stent graft segment or the distal-side stent graft segment connected to the first positioning stent is parallel to the surface of the positioning stent. Further, the first positioning stent may be a bare stent, tips thereof are designed to be an inwardly-retracted shape so as to prevent the tips from puncturing an aortic wall, and the first positioning stent is mainly used for releasing the first stent body in vivo. The first positioning stent may be provided with barbs, so as to prevent the first stent body from being displaced after being impacted by the blood flow in the aorta. Similarly, barbs may also be provided at the first end of the proximal-side stent graft segment or the second end of the distal-side stent graft segment.

Preferably, the occlusion membrane further comprises a second opening, and the second opening of the occlusion membrane in the proximal-side stent graft segment and the second opening of the occlusion membrane in the distal-side stent graft segment are in communication with each other through an aortic channel.

Preferably, the first opening is provided on one side of the occlusion membrane, and the second opening is provided on the other side of the occlusion membrane.

Preferably, the second opening is provided in the middle position of the occlusion membrane, and the first openings are provided on both sides of the second opening.

Preferably, the distal end of the distal-side stent graft segment is provided with an extension section, the extension section being internally provided with two second stent graft channels provided in parallel; one part of each of the second stent graft channels is provided in the extension section, and the other part thereof extends out of the extension section and is in communication with a corresponding branch artery stent graft, and the two second stent graft channels fill the extension section. When the present disclosure is used for a patient whose aortic lesions extend to the abdominal aorta region, it is also necessary to simultaneously complete the connection between the first stent body and arteria iliaca communis; and therefore, some embodiments of the present disclosure further provide that at least one second stent graft channel is provided in the proximal-side stent graft segment or the distal-side stent graft segment; wherein one part of the second stent graft channel is provided in the proximal-side stent graft segment or the distal-side stent graft segment, and the other part thereof extends out of the end of the proximal-side stent graft segment or the distal-side stent graft segment away from the bare stent segment and is in communication with a corresponding artery (such as an iliac artery).

Preferably, the cross sections of the two second stent graft channels form a double D-shape.

When the described branch artery reconstruction assist device is used to reconstruct a branch artery channel, in addition to flowing into branch arteries through the first opening, blood flow may also flow freely in and out of the first stent body through the second opening; in this case, the aortic lesions are not isolated from the blood flow, and the blood flow may also flow freely out of the first stent body. That is to say, when the branch artery reconstruction assist device provided according to some embodiments of the present disclosure performs branch artery-first aortic reconstruction, blood can flow from the upstream of the aorta to the downstream of the aorta through the first stent body, the human body does not have the risk of ischemia, and the blood in the artery is also not isolated from the lesion sites.

Some embodiments of the present disclosure further provide an S-C branch-first aortic reconstruction system, comprising the branch artery reconstruction assist device in any one above and a second stent body; wherein the second stent body is a stent graft for forming an aortic channel, and is used for being provided within a first stent body after completing positioning and releasing of the first stent body and completing the reconstruction of branch arteries, to form the aortic channel, so as to communicate with blood flow within the aorta while isolating lesion sites. In the process of reconstruction of branch arteries, the blood flow flows through the occlusion membrane at a blood-facing end of the first stent body through the first opening, enters the first stent graft channel, then flows into a branch artery where the blood flow is located through a corresponding branch artery stent graft; meanwhile the blood also flows in or out of the first stent body through the second opening (without being isolated from lesion sites). It should be understood that, the first stent graft channel may be provided through the first opening, and the position where the first stent graft channel is provided through the first opening may be any position, for example, two ends in an extension (i.e. length) direction of the first stent graft channel, or any position between the two ends.

Preferably, two ends of the second stent body are respectively in communication with the second opening on the occlusion membrane in the proximal-side stent graft segment and the second opening on the occlusion membrane in the distal-side stent graft segment.

Preferably, a leakproof channel is provided in the proximal-side stent graft segment and/or the distal-side stent graft segment, wherein one end of the leakproof channel is connected to the second opening, and the other end of the leakproof channel is connected to an end corresponding to the second stent body. Specifically, one end of the leakproof channel is connected to the second opening, and the other end of the leakproof channel is connected to the second stent body. When the leakproof channel is connected to the second stent body, the leakproof channel is preferably sleeved with the second stent body, that is, the leakproof channel is connected to the second stent body in an overlapping manner. After the branch artery accesses are reconstructed by using the first stent graft channel, the second stent body is then placed in the first stent body, two ends of the second stent body are connected to leakproof channels, the blood flow flows into the second stent body from the leakproof channel at one end of the first stent body, and then flows into the other end of the first stent body until the blood flow flows out of the first stent body; in this case, the branch-first aortic reconstruction system provided in some embodiments of the present disclosure smoothly reconstructs the aortic access of the body. It is worth mentioning that, in this case, the blood flow is isolated from lesion sites in branch artery regions, and the blood flow can flow from the upstream aorta into the branch arteries through the first stent graft channel and flow into the downstream aorta through the second stent body.

Preferably, an end of the second stent body is provided with a second positioning stent, and the second stent body is connected to an end of the leakproof channel by means of the second positioning stent. In order to facilitate anchoring of the second stent body to the first stent body, barbs are provided at one end or two ends of the second stent body, which can be understood as providing first barbs at the proximal side of the second stent body to fasten the second stent body to the first stent body, and also providing second barbs at the distal side of the second stent body. Alternatively, only the proximal side of the second stent body may be provided with first barbs. The second stent body can be allowed to be fixed on the inner wall of the leakproof channel of the first stent body of the vessels under the impact of high-speed and high-pressure blood flow of the aorta. Here, similar to the first stent body, a positioning stent is also provided at one end of the second stent body, and is used for connecting to a stent conveyer, so as to facilitate conveying the second stent body to a predetermined position. Herein, the diameter of the second stent body after release is equal to or greater than the inner diameter of the leakproof channel, so as to improve the sealing performance between the second stent body and the leakproof channel.

Preferably, said reconstruction system further comprises a branch artery stent graft connected to the first stent graft channel and/or the second stent graft channel.

When the first stent body and the second stent body are used cooperatively, only the first stent body is provided with a positioning mark, or only the second stent body is provided with a positioning mark, or the first stent body and the second stent body are each provided with a positioning mark. The positioning mark is used for positioning under X-ray to assist in determining the position of the stent during surgery. The positioning mark can be arranged at any position of the first stent body, for example, at the distal side. The positioning mark may also be located on all positions of the first stent body. Similarly, the positioning mark may be arranged at any position of the first stent body, for example, at the distal side. The positioning mark may also be located on all positions of the first stent body.

Preferably, the diameter of the branch artery stent graft is gradually changed; more preferably, the diameter of the end of the branch artery stent graft connected to the first stent graft channel or the second stent graft channel is smaller than the diameter of the other end of the branch artery stent graft channel; or the diameter of the end of the branch artery stent graft connected to the first stent graft channel or the second stent graft channel is greater than the diameter of the other end of the branch artery stent graft channel. It should be noted that the gradually-changing diameter is not limited to the situations above, and is dependent on specific requirements in clinical practice.

It can be determined from the illustration above that the branch artery reconstruction assist device provided in some embodiments of the present disclosure can place the first stent body in vivo while blood in the aorta is unobstructed to complete the step of branch-first reconstruction; whereas in the S-C branch-first aortic reconstruction system, by combined use of the first stent body and the second stent body, first, the first stent body is provided in the aorta, and an aorta-branch blood flow channel is constructed by means of embedded stents and branch artery stent grafts provided in branch arteries, thereby achieving the step of branch-first reconstruction (branch artery-first reconstruction can solve, to a certain extent, the problems of complex structure, blood leakage and low patency, etc. commonly existing in stents for treating multi-branch aortic diseases). After the branch artery accesses are reconstructed, the second stent body is then sleeved on the leakproof channel of the first stent body to jointly construct an aorta-aorta blood flow channel. The second stent body and the first stent body achieve sealing cooperation to isolate lesions, endoleak does not occur in the stents and various arteries are unobstructed, and normal supply of cerebral blood flow and/or organ blood flow during surgery is achieved, thereby avoiding vascular exclusion, and further reducing complications caused by ischemia.

With respect to the related art, advantages of some embodiments of the present disclosure lie in:
1. Some embodiments of the present disclosure are used for isolating aortic dissection or aneurysm lumen, which can avoid endoleak.
2. Branch reconstruction and lesion isolation during surgery are divided into two steps: first, the first stent body is used to construct blood flow of three branch arteries of an aortic arch segment or four branch arteries of visceral artery branch vessels of a human body in a thoracoabdominal aorta, and then the second stent body is implanted to isolate lesions of the aortic arch segment or the thoracoabdominal aorta, which allow to achieve the goal of the necessity of maintenance of blood flow during surgery of reconstructing branch vessels, thereby avoiding ischemia.
3. In some embodiments of the present disclosure, branch channels are constructed in an open state without ischemia, thereby avoiding complicated steps such as pre-fenestration on a stent graft or in-situ fenestration after implantation in the related art, and avoiding organ ischemia caused by long-term surgery.

### Brief Description of the Drawings

In order to describe the technical solutions in some embodiments of the present disclosure or in the related art more clearly, hereinafter, accompanying drawings requiring to be used in the embodiments or the related art will be introduced briefly. Apparently, the accompanying drawings in the following description merely relate to embodiments of the present disclosure, and for a person of ordinary skill in the art, other accompanying drawings can also be obtained according to these accompanying drawings without involving any inventive effort.
Fig. 1a is a schematic structural diagram of a first stent body in an S-C branch-first aortic arch reconstruction system before use according to embodiments of the present disclosure;
Fig. 1b is a schematic structural diagram of a second stent body in an S-C branch-first aortic arch reconstruction system before use according to embodiments of the present disclosure;
Fig. 2 is a schematic structural diagram of combined use of a first stent body and a second stent body in an S-C branch-first aortic arch reconstruction system according to embodiments of the present disclosure;
Fig. 3 is a schematic structural diagram of a first stent body in an S-C branch-first aortic arch reconstruction system being provided in an aortic arch according to embodiments of the present disclosure;
Fig. 4a is a schematic sectional diagram of a first stent graft segment of a first stent body in an S-C branch-first aortic arch reconstruction system according to embodiments of the present disclosure;
Fig. 4b is a schematic diagram of a proximal-side part of a first stent graft segment of a first stent body in an S-C branch-first aortic arch reconstruction system according to embodiments of the present disclosure;
Fig. 5a is a schematic sectional diagram of a second stent graft segment of a first stent body in an S-C branch-first aortic arch reconstruction system according to embodiments of the present disclosure;
Fig. 5b is a schematic diagram of a distal-side part of a second stent graft segment of a first stent body in an S-C branch-first aortic arch reconstruction system according to embodiments of the present disclosure;
Fig. 6 is a schematic structural diagram of a distal side after combined use of a first stent body and a second stent body in an S-C branch-first aortic arch reconstruction system according to embodiments of the present disclosure;
Fig. 7a is a schematic structural diagram of a first-type first stent body in an S-C branch-first thoracoabdominal aortic reconstruction system before use according to embodiments of the present disclosure;
Fig. 7b is a schematic structural diagram of a second stent body in an S-C branch-first thoracoabdominal aortic reconstruction system before use according to embodiments of the present disclosure;
Fig. 7c is a schematic diagram of a second-type first stent body in an S-C branch-first thoracoabdominal aortic reconstruction system before use according to embodiments of the present disclosure;
Fig. 7d is a schematic diagram of a third-type first stent body in an S-C branch-first thoracoabdominal aortic reconstruction system before use according to embodiments of the present disclosure;
Fig. 8a is a schematic structural diagram of a first-type first stent body in an S-C branch-first thoracoabdominal aortic reconstruction system being provided in a thoracoabdominal aorta according to embodiments of the present disclosure;
Fig. 8b is a schematic structural diagram of combined use of a first-type first stent body and a second stent body in an S-C branch-first thoracoabdominal aortic reconstruction system according to embodiments of the present disclosure;
Fig. 8c is a schematic structural diagram of a second-type first stent body in an S-C branch-first thoracoabdominal aortic reconstruction system being provided in a thoracoabdominal aorta according to embodiments of the present disclosure;
Fig. 8d is a schematic structural diagram of combined use of a second-type first stent body and a second stent body in an S-C branch-first thoracoabdominal aortic reconstruction system according to embodiments of the present disclosure;
Fig. 8e is a schematic structural diagram of a third-type first stent body in an S-C branch-first thoracoabdominal aortic reconstruction system being provided in a thoracoabdominal aorta according to embodiments of the present disclosure;
Fig. 8f is a schematic structural diagram of combined use of a third-type first stent body and a second stent body in an S-C branch-first thoracoabdominal aortic reconstruction system according to embodiments of the present disclosure;
Fig. 9a is a schematic structural diagram of a proximal side of a first-type first stent body in an S-C branch-first thoracoabdominal aortic reconstruction system according to embodiments of the present disclosure;
Fig. 9b is a schematic sectional diagram of Fig. 9a;
Fig. 10a is a schematic structural diagram of a distal side of a first-type first stent body in an S-C branch-first thoracoabdominal aortic reconstruction system according to embodiments of the present disclosure;
Fig. 10b is a schematic sectional diagram of Fig. 10a;
Fig. 11a is another schematic structural diagram of a distal side of a second stent graft segment in an S-C branch-first thoracoabdominal aortic reconstruction system according to embodiments of the present disclosure; and
Fig. 11b is a schematic sectional diagram of Fig. 11a.

### Detailed Description of the Embodiments

In order to make objects, technical solutions and advantages of some embodiments of the present disclosure clearly understood, hereinafter, some embodiments of the present disclosure will be further described in detail in conjunction with specific embodiments and with reference to the accompanying drawings.

It should be noted that, unless defined otherwise, technical terms or scientific terms used in embodiments of the present disclosure shall have common meanings as understood by a person of ordinary skill in the art to which the present disclosure belongs. The terms "first", "second", and similar words used in the embodiments of the present disclosure do not indicate any sequence, number, or importance, but are only used to distinguish different constituent parts. Similar words such as "comprising" or "including" and the like mean that the element or item appearing before the word encompasses elements or items listed after the word and equivalents thereof, without excluding other elements or items. Similar words such as "connected" or "connected to" and the like are not limited to physical or mechanical connections, but may comprise electrical connections, whether direct or indirect. The terms "upper", "lower", "left", "right" and the like are only used for representing the relative position relationship, and when the absolute position of an object to be described changes, the relative position relationship may also change correspondingly.

In addition, numerical values mentioned in the embodiments of the present disclosure are merely examples, and are not intended to limit the technical features of some embodiments of the present disclosure.

Endovascular treatment can achieve minimally-invasive repair that does not open the chest and does not block blood flow by implantation of a stent graft (for example, implantation of a fenestration stent, implantation of a branch artery stent graft, a chimney technology, and the like). However, the surgery difficulty is very high as the treatment achieve both isolation of lesions and maintenance of vascular patency. To this end, an S-C branch-first aortic reconstruction system provided in some embodiments of the present disclosure can achieve both isolation of lesions and maintenance of vascular patency, and specifically comprises a branch artery reconstruction assist device and a second stent body, wherein the branch artery reconstruction assist device comprises a first stent body. It should be understood that, during a "branch-first" aortic and branch artery reconstruction surgery, it is necessary to first use the branch artery reconstruction assist device to reconstruct branch artery accesses, and after the branch artery accesses are reconstructed, the second stent body is used as needed to reconstruct an aortic access.

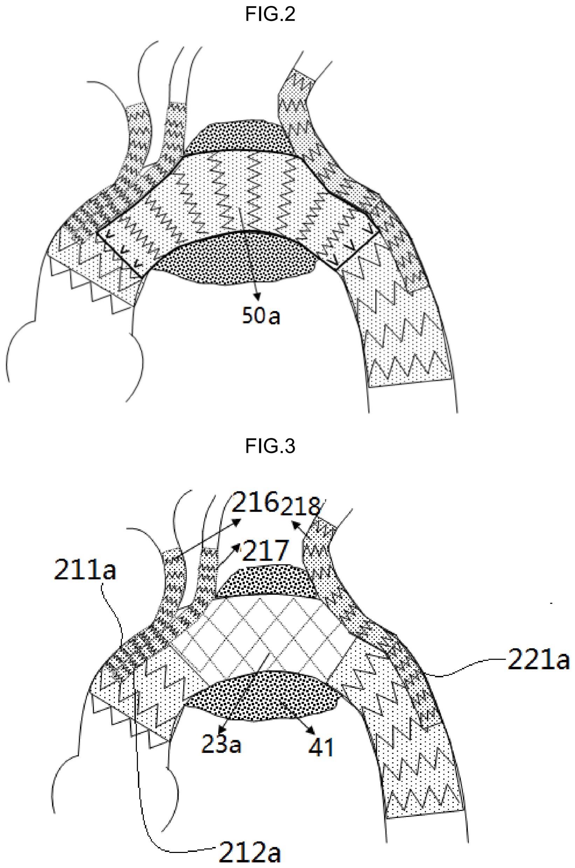

In the embodiments of the present disclosure, detailed illustration is made by taking the S-C branch-first aortic reconstruction system being used in an aortic arch segment and some distal ascending aortic lesions or thoracoabdominal aortic lesions as an example, but some embodiments of the present disclosure are not limited to be used at the aortic arch and the thoracoabdominal aorta. When used in other aortic lesions, it is only necessary to adjust the positions and the number of first stent graft accesses in a proximal-side stent graft segment (equivalent to a first stent graft segment) and a distal-side stent graft segment (equivalent to a second stent graft segment) correspondingly according to the positions and the number of branch arteries, and the technical effects of preventing endoleak and ischemia in some embodiments of the present disclosure can still be achieved. In some embodiments of the present disclosure, the proximal side may be understood as the end that is proximal to the heart (e.g. the direction in which blood flows in as shown in Fig. 2a-Fig. 2c), and the distal side may be understood as the end that is relatively distal to the heart (e.g. the direction in which blood flows out as shown in Fig. 2).

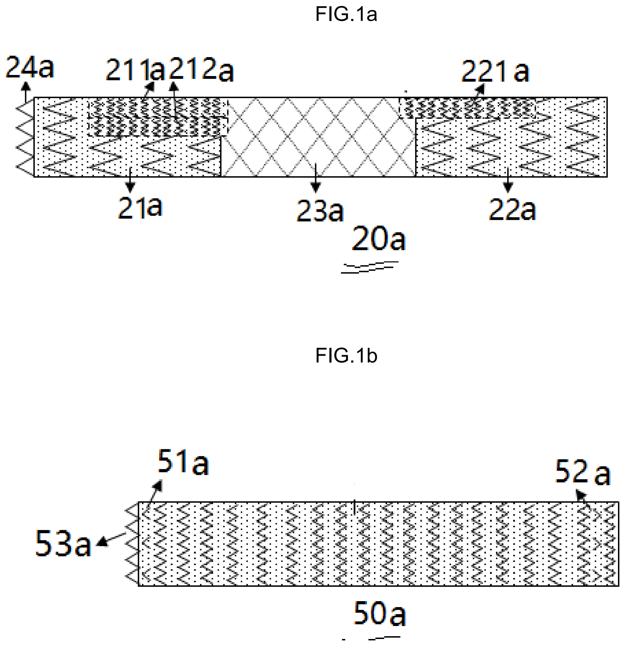

In some embodiments, the S-C branch-first aortic reconstruction system provided in some embodiments of the present disclosure is an S-C branch-first aortic arch reconstruction system, wherein the aorta is an aortic arch segment, and branch arteries thereof are respectively an innominate artery, a common carotid artery and a subclavian artery. It should be understood that, regarding the first stent body 20a and the second stent body 50a, before the aortic access is reconstructed, the first stent body 20a and the second stent body 50a are separated, as shown in Fig. 1a and Fig. 1b; whereas when the aorta is reconstructed, the first stent body 20a and the second stent body 50a are used cooperatively, as shown in Figs. 2 and 3. After being implanted into an aortic arch segment, the first stent body 20a and the second stent body 50a both ride across the aortic arch segment in a saddle shape, and the stability of the combination thereof is much higher than that of other arch branch artery stent graft systems.

Hereinafter, the branch artery reconstruction assist device is first described in detail. The branch artery reconstruction assist device comprises a first stent body; the first stent body comprises a middle bare stent segment, and a proximal-side stent graft segment and a distal-side stent graft segment connected to two ends of the bare stent segment, and the branch artery reconstruction assist device is provided at positions of the aorta and corresponding branch arteries.

As shown in Fig. 1a, in this embodiment, the branch artery reconstruction device comprises a first stent body 20a; the first stent body 20a may have a hollow lumen, and the first stent body 20a comprises a bare stent segment 23a and a proximal-side stent graft segment 21a and a distal-side stent graft segment 22a that are connected to two ends of the bare stent segment. Before the S-C branch-first aortic reconstruction system provided in some embodiments of the present disclosure is used, blood flows in the arteries of the human body, and after the first stent body 20a is released in vivo and in vivo positioning is completed, the blood can flow in the first stent body 20a; that is, the blood flows from the proximal side of the first stent body 20a, passes through the bare stent segment 23a, and flows to the distal side of the first stent body 20a; and also that is, the blood flows from a first end of the proximal-side stent graft segment 21a toward a second end of the distal-side stent graft segment 22a. Here, the bare stent 23a has a bendable structure, so as to adapt to different aortic structures.

The first end herein may be provided at any position of the proximal-side stent graft segment 21a, for example, at the distal end of the proximal-side stent graft segment 21a; and the second end herein may be provided at any position of the distal-side stent graft segment 22a, such as the distal end of the distal-side stent graft segment 22a, so as to prevent blood from forming thrombus in the first stent body 20a. The first stent body 20a is an integral structure, that is, the proximal-side stent graft segment 21a, the bare stent segment 23a, and the distal-side stent graft segment 22a form an integral structure.

Herein, the proximal-side stent graft segment 21a and the distal-side stent graft segment 22a are stent grafts, and the bare stent segment 23a is bare stent. When the first stent body 20a is used for an aortic arch, all three branch arteries in the arch segment are not covered by a covering membrane, therefore are in an ischemia-free state, branch stent grafts linked to the first stent body 20a are respectively embedded in an innominate artery, a left common carotid artery and a left subclavian artery, as shown in Fig. 3. That is, the positions of an innominate artery branch artery stent graft 216, a common carotid artery branch artery stent graft 217, and a subclavian artery branch artery stent graft 218 relative to the bare stent segment 23a generally coincide with the positions of openings, in the aortic arch, of the innominate artery, the left common carotid artery and the left subclavian artery in the branch arteries on the aortic arch, respectively.

Further, each of the proximal-side stent graft segment and the distal-side stent graft segment is provided with an occlusion membrane and at least one first stent graft channel; the occlusion membrane comprises at least one first opening, and the first opening is correspondingly connected to a first end of the first stent graft channel, and a second end of the first stent graft channel is connected to a corresponding branch artery stent graft to form a branch channel so as to pass through the bare stent. Here, the occlusion membrane further comprises a second opening, and the second opening of the occlusion membrane in the proximal-side stent graft segment and the second opening of the occlusion membrane in the distal-side stent graft segment are in communication with each other through an aortic channel.

The first opening and the second opening herein may be provided on the occlusion membrane as required. For example, in some embodiments, the first opening is provided on one side of the occlusion membrane, and the second opening is provided on the other side of the occlusion membrane. For example, in some other embodiments, the second opening is provided in the middle position of the occlusion membrane, and the first openings are provided on both sides of the second opening.

In some specific embodiments, a total of three first stent graft channels are provided in the proximal-side stent graft segment 21a and the distal-side stent graft segment 22a (namely, a different number of first stent graft channels can be provided thereon according to the surgical habits of different doctors), and the first stent graft channels are respectively in communication with branch artery stent grafts in the innominate artery, the common carotid artery and the subclavian artery; wherein the branch artery stent grafts pass for example, through wire mesh gaps of the bare stent segment 23a. By the same reasoning, it may be also configured such that the first stent graft channels pass through the wire mesh gaps of the bare stent segment 23a and are respectively in communication with branch artery stent grafts in the innominate artery, the common carotid artery and the subclavian artery. In this embodiment, two first stent graft channels are provided in the proximal-side stent graft segment 21a, and one first stent graft channel is provided in the distal-side stent graft segment 22a. It is well understood by a person skilled in the medical field that the first stent graft channel on the distal-side stent graft segment 22a enables blood flow to flow into the channel and flow to corresponding branch arteries by means of a differential pressure.

The first stent graft channels may be formed by corresponding embedded stent grafts (211a, 212a, 221a); wherein each of the embedded stent grafts may comprise an embedded stent body having a lumen and a covering membrane provided outside the embedded stent body. That is, the first stent graft channel is formed by a lumen of a corresponding embedded stent graft. In some embodiments, two embedded stent grafts, i.e. a first embedded stent graft 211a and a second embedded stent graft 212a, may be provided in the proximal-side stent graft segment 21a; and one embedded stent graft, i.e. a third embedded stent graft 221a, may be provided in the distal-side stent graft segment 22a.

When the first stent body 20a is provided within the aortic arch, branch arteries (i.e. branch vessels) are reconstructed by the first embedded stent graft 211a, the second embedded stent graft 212a and the third embedded stent graft 221a, and thereafter the second stent body 50a is placed into the first stent body 20 in a matching manner, so as to construct the aortic channel and isolate lesions at the aortic arch. In this way, the first stent body 20a and the second stent body 50a are used in cooperation to achieve a branch-first reconstruction step, such that branch arteries are unobstructed during surgery, and cerebral blood flow is normally supplied, thereby avoiding vascular exclusion, and further avoiding cerebral ischemia, thereby reducing the incidence rate of cerebral infarction.

Further, the distance between an inlet end of a branch artery corresponding to the proximal-side stent graft segment 21a and an inlet end of a branch artery corresponding to the distal-side stent graft segment 22a is smaller than the length of the bare stent 23a, such that a second end of the first stent graft channel and a corresponding branch artery stent graft are connected to form a branch channel so as to pass through the bare stent 23a.

In some embodiments, in a radial direction, the size (i.e. diameter) of the first embedded stent graft 211a is slightly less than the deployment size of the innominate artery branch artery stent graft in a first overlapping region. The size of the second embedded stent graft 212a is slightly less than the deployment size of the common carotid artery branch artery stent graft 217 in a second overlapping region. The size of the third embedded stent graft 221a is slightly less than the deployment size of the subclavian artery branch artery stent graft 218.

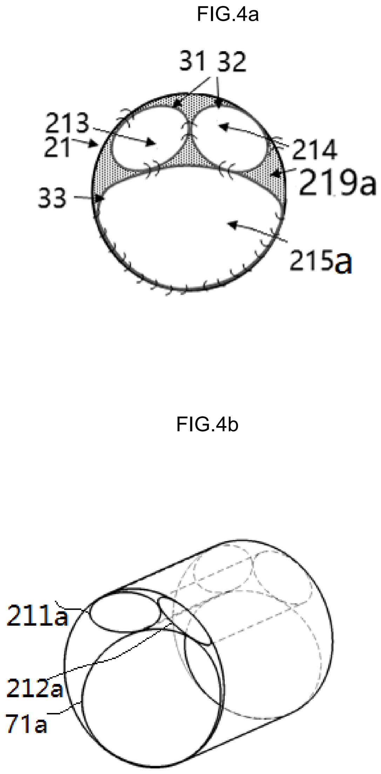

In some embodiments, the shapes of the first embedded stent graft 211a and the second embedded stent graft 212a may be elliptical or circular, as shown in Fig. 4a, as long as the sizes of the first embedded stent graft 211a and the second embedded stent graft 212a are adapted to branch arteries to which they are to be implanted.

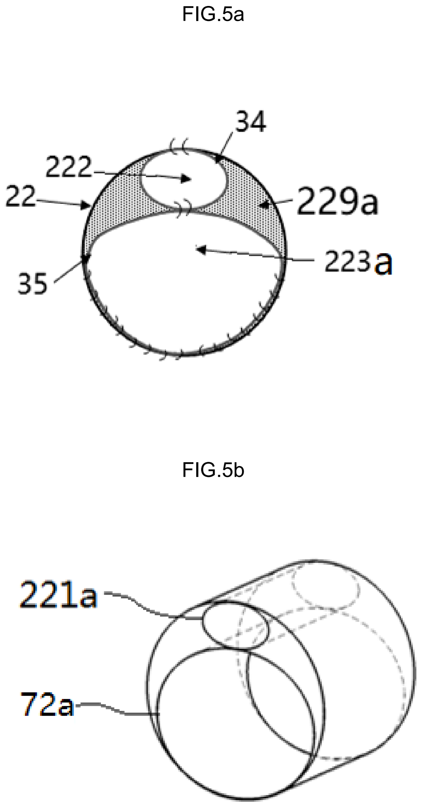

In some embodiments, the shape of the third embedded stent graft 221a may be circular or elliptical, as shown in Fig. 5a.

In some embodiments, the lengths of the embedded first embedded stent graft 211a, second embedded stent graft 212a, and third embedded stent graft 221a need to be sufficient to be in nested connection with the branch artery stent grafts, so as to avoid the occurrence of endoleak due to an excessively short overlapping region. These embedded stent grafts can be partially extruded into the bare stent 23a, and branch artery stent grafts such as the left subclavian artery branch artery stent graft 218 among branch arteries such as the corresponding left subclavian artery can be well provided in these embedded stent grafts, without slits, and can be sufficiently attached to the inner walls of the embedded stent grafts to ensure no leakage of blood. Each branch artery stent graft enters the bare stent segment 23a from the wire mesh gaps of the bare stent segment 23a, and is connected to the embedded stent graft.

It should be understood that, the innominate artery branch artery stent graft may adopt an existing branch artery stent graft, as long as they can be implanted into a corresponding artery branch.

In some embodiments, the distal side of the first embedded stent graft 211a is coaxially nested with the proximal side of the innominate artery branch artery stent graft; and the distal side of the first embedded stent graft 211a has a first overlapping region nested with the proximal side of the corresponding innominate artery branch artery stent graft. The size of the first overlapping region in an axial direction (i.e. the length of the first overlapping region) is greater than preset 30 mm, and may also be greater than other preset values, and 30 mm is merely taking an example. Similarly, each of the distal side of the second embedded stent graft 212a and the distal side of the third embedded stent graft 221a is also coaxially nested correspondingly, and a preset value is set in the overlapping region, such that after the branch artery stent graft is released in the embedded stent graft, there is sufficient contact surface between the branch artery stent graft and the embedded stent graft to achieve firm connection with each other.

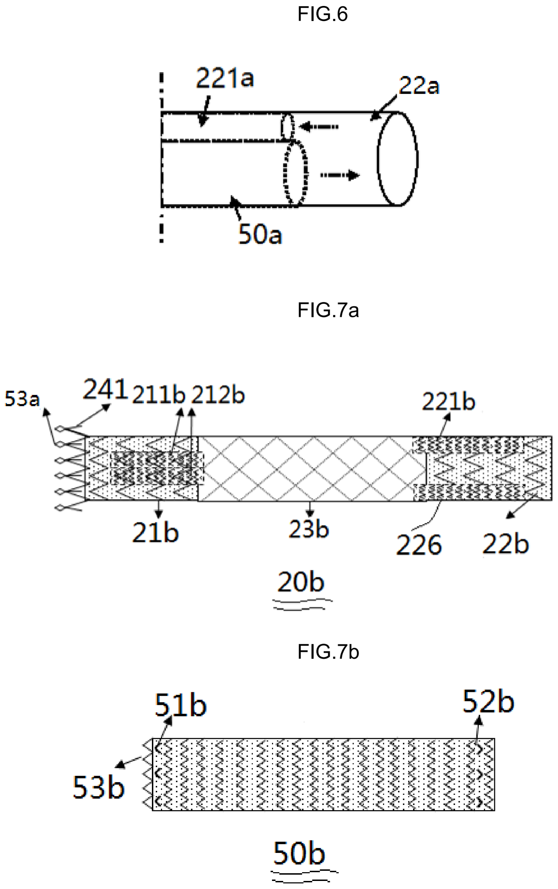

In some embodiments, as shown in Fig. 6, the length of the distal-side stent graft segment 22a is larger than an overlapping portion between same and the third embedded stent graft 221a, which not only ensures that the second stent body 50a has sufficient overlapping of stent graft segment with the first stent body 20a after being implanted into the first stent body 20a on the aortic arch, but also does not cover the distal side of the embedded stent graft (i.e. the third embedded stent graft 221a) in a tail-end covering membrane portion (i.e. the distal-side stent graft segment 22a) of the first stent body 20a. Moreover, when the reconstruction system is used in the thoracoabdominal aorta, when the distal side of the first stent body 20a needs to extend the stent graft (i.e. extending the distal-side stent graft segment 22a), an opening of the embedded stent graft (i.e. the third embedded stent graft 221a) will not be covered.

In some embodiments, occlusion membranes (219a, 229a) are respectively provided in the proximal-side stent graft segment 21a and the distal-side stent graft segment 22a. Specifically, a proximal-side occlusion membrane 219a (hereinafter referred to as a proximal-side occlusion membrane 219a) is provided in the proximal-side stent graft segment 21a, and a distal-side occlusion membrane 229a (hereinafter referred to as a distal-side occlusion membrane 229a) is provided in the distal-side stent graft segment 22a. The proximal-side occlusion membrane 219a may be provided at any position in the proximal-side stent graft segment 21a, and the distal-side occlusion membrane 229a may be provided at any position in the distal-side stent graft segment 22a. It is only necessary to ensure that the placement position of the first stent body 20a after surgery can make a lesion site located between the proximal-side occlusion membrane 219a and the distal-side occlusion membrane 229a, and blood flow in the aorta of a patient does not contact the lesion site.

In some embodiments, the proximal-side occlusion membrane 219a is a membrane formed by sealing and suturing one circle on the covering membrane of the proximal-side stent graft segment 21a along the circumferential direction of the proximal-side stent graft segment 21a; and the proximal-side occlusion membrane 219a prevents, from the radial direction of the first stent body 20a, blood flow in the aorta from prevent to flow into the gaps between channels in 20a and the suture position may be on the inner wall or the outer wall of the proximal-side stent graft segment 21a.

Specifically, the proximal-side occlusion membrane 219a is provided with a first opening 213, a first opening 214 and a second opening 215a, as shown in Fig. 4a; the blood flow cannot flow into the first stent body 20a from the shadow parts, and can only enter the first opening 213, the first opening 214 and the second opening 215a. An edge of the first opening 213 is sutured to one end of the first embedded stent graft 211a, and the other end of the first embedded stent graft 211a is embedded with the innominate artery branch artery stent graft 216 to form a reconstructed blood flow channel of an innominate artery branch vessel. An edge of the first opening 214 is sutured to one end of the second embedded stent graft 212a, and the other end of the second embedded stent graft 212a is embedded with the common carotid artery branch artery stent graft 217 to form a reconstructed blood flow channel of a common carotid artery branch vessel.

Further, the occlusion membrane in the proximal-side stent graft segment is connected to the proximal end of the proximal-side stent graft segment, and the occlusion membrane in the distal-side stent graft segment is connected to a distal end of the distal-side stent graft segment.

In some embodiments, the distal-side occlusion membrane 229a is provided in the distal-side stent graft segment 22a in the radial direction, and the distal-side occlusion membrane 229a is attached to the inner wall of the distal-side stent graft segment 22a and is provided with a first opening 222 and a second opening 223, as shown in Fig. 5a and Fig. 5b. The first opening 222 is connected to the third embedded stent graft 221a which is nested with the subclavian artery branch artery stent graft 218, so as to form a reconstructed blood flow channel of a subclavian artery branch vessel. The second stent body 50a is nested in the second opening 223, and the second stent body 50a will be described in detail below.

In the present embodiment, considering that each of the proximal-side stent graft segment and the distal-side stent graft segment is provided an occlusion membrane; the occlusion membrane comprises a first opening, and the first opening is correspondingly connected to a first end of the first stent graft channel; wherein when the connection position between the proximal-side stent graft segment or the distal-side stent graft segment and the occlusion membrane is flush with the first end of the first stent graft channel, the occlusion membrane is in a planar shape; and/or when the connection position between the proximal-side stent graft segment or the distal-side stent graft segment and the occlusion membrane is not flush with the first end of the first stent graft channel, the occlusion membrane is in an inclined surface shape.

Specifically, when the connection position between the proximal-side stent graft segment 21a or the distal-side stent graft segment 22a and the occlusion membrane is flush with the first end of the first stent graft channel, the occlusion membrane is in a planar shape; and/or when the connection position between the proximal-side stent graft segment 21a or the distal-side stent graft segment 22a and the occlusion membrane is not flush with the first end of the first stent graft channel, the occlusion membrane is in an inclined surface shape.

Herein, the connection position between the occlusion membrane and the proximal-side stent graft segment 21a or the distal-side stent graft segment 22a may be set according to the relative position between the first stent graft channel and the proximal-side stent graft segment 21a or the distal-side stent graft segment 22a, so as to adjust the shape of the occlusion membrane.

In some embodiments, for example, when the first end of the first stent graft channel is flush with the proximal-side edge of the proximal-side stent graft segment 21a, the proximal-side occlusion membrane 219a is connected to the end of the proximal-side stent graft segment 21a, such that the proximal-side occlusion membrane 219a is in a planar shape; or so to speak, the first end of the first stent graft channel is arranged in parallel to the radial direction of the proximal-side stent graft segment 21a, in which the first openings (213, 214) are in the plane where the proximal-side end point of the proximal-side stent graft segment 21a is located. When the first end of the first stent graft channel is located inside the proximal-side stent graft segment 21a, the proximal-side occlusion membrane 219a is connected to an end of the proximal-side stent graft segment 21a, such that the proximal-side occlusion membrane 219a is in an inclined surface shape, e.g. in a funnel shape as shown in Fig. 4b, and the first openings (213, 214) are in the proximal-side stent graft segment 21a.

Similarly, when the first end of the first stent graft channel is arranged flush with the distal-side edge of the distal-side stent graft segment 22a, the second occlusion membrane 220a is connected to the end of the distal-side stent graft segment 22a, such that the distal-side occlusion membrane 229a is in a planar shape; and when the first end of the first stent graft channel is located inside the distal-side stent graft segment 22a, the second occlusion membrane 220a is connected to the end of the distal-side stent graft segment 22a, such that the distal-side occlusion membrane 229a is funnel-shaped. It should be understood that the shapes of the proximal-side occlusion membrane 219a and the distal-side occlusion membrane 229a are independent from each other, and may be the same or different.

In some embodiments, the first embedded stent graft 211a and the second embedded stent graft 212a are respectively attached to and provided within the proximal-side stent graft segment 21a and are adjacent to each other, and one end of the first embedded stent graft 211a and one end of the second embedded stent graft 212a are flush with the proximal side of the proximal-side stent graft segment 21a. The third embedded stent graft 221a is attached to and provided within the distal-side stent graft segment 22a.

Further, a first positioning stent is provided at the proximal end of the proximal-side stent graft segment and/or at the distal end of the distal-side stent graft segment. In the S-C branch-first aortic arch reconstruction system provided in embodiments of the present disclosure, a part of the first stent body is three parts integrated as a whole. A part of the proximal-side stent graft segment 21a is a stent graft and is internally embedded with channels of two sub-stent grafts (for example, the first embedded stent graft 211a and the second embedded stent graft 212a). A first positioning stent 24a is provided at a front end (i.e. the proximal end) of the proximal-side stent graft segment 21a, and tips of the first positioning stent 24a are designed to be an inwardly-retracted shape so as to prevent the tips from puncturing an ascending aortic wall. A part of the distal-side stent graft segment 22a is a stent graft and is internally embedded with a channel of one sub-stent graft (for example, the third embedded stent graft 221a), and a part of the bare stent segment 23a connected in the middle is an arched bare stent.

In some embodiments, the first positioning stent 24a is further provided with barbs 241 (not shown in Fig. 1a) for better anchoring the first stent body to the inner walls of vessels. Similarly, barbs 241 (not shown in Fig. 1a) may also be provided at the end of the proximal-side stent graft segment or the distal-side stent graft segment.

In some embodiments, the second stent body 50a is a stent graft, and is configured to be implanted into the first stent body 20a and placed in parallel with the first stent graft channels at two ends of the first stent body (i.e. the proximal-side stent graft segment 21a and the distal-side stent graft segment 22a) after the first stent body is provided in the aortic arch (or the thoracoabdominal aorta) and branch artery reconstruction is completed, so as to cooperate with the first stent body 20a to isolate lesion sites of the aorta.

Further, a leakproof channel is provided in the proximal-side stent graft segment and/or the distal-side stent graft segment, wherein one end of the leakproof channel is connected to the second opening, and the other end of the leakproof channel is connected to an end corresponding to the second stent body.

In some embodiments, the occlusion membrane is provided with the second openings (215, 223), such that the blood flow flows from the second opening 215 to the bare stent segment 23a and flows out of the second opening 223 from the bare stent segment 23a. Further, the proximal side of the second stent body 50a may be connected to the second opening 215, and the distal side of the second stent body 50a may be connected to the second opening 223, such that the first stent body 20a isolates lesion sites 41 of the aortic arch or lesion sites of the thoracoabdominal aorta. Further, in order to facilitate detachable placement of the first stent body 20a in the second stent body 50a, a leakproof channel is provided in each of the proximal-side stent graft segment 21a and the distal-side stent graft segment 22a, wherein one end of the leakproof channel is connected to the second opening, and the other end of the leakproof channel is connected to the second stent body 50a. That is, the second stent body 50a is configured to be in communication with the branch arteries by means of the first stent body 20a and then to be provided in the bare stent segment 23a, so as to be in communication with the blood flow in the aorta.

In some embodiments, a first leakproof channel 71a is provided in the proximal-side stent graft segment 21a, and a second leakproof channel 72a is provided in the distal-side stent graft segment 22a. In this way, the second stent body 50a is detachably nested in the first leakproof channel 71a and the second leakproof channel 72a. One end (for example, the proximal side) of the first leakproof channel 71a is connected to the second opening 215 on the proximal-side occlusion membrane 219a, and the other end (for example, the distal side) of the first leakproof channel 71a abuts against the outer wall of the second stent body 50a. One end (for example, the distal side) of the second leakproof channel 72a is connected to the second opening 223 on the distal-side occlusion membrane 229a, and the other end (for example, the proximal side) of the second leakproof channel 72a abuts against the outer wall of the second stent body 50a.

In some embodiments, the proximal side of the embedded stent graft in the proximal-side stent graft segment 21a does not exceed the proximal side of the second stent body 50a, as shown in Fig. 6, the linear distance between the proximal side of the third embedded stent graft 221a and the proximal side of the second stent body 50a does not exceed 10 mm. Similarly, the distance between the distal side of the embedded stent in the distal-side stent graft segment 22a and the distal side of the second stent body 50a may also be set to be within 10 mm. In this way, it can be ensured that the two-stent system can achieve firm attachment without endoleak.

In some embodiments, the second stent body 50a is a stent graft. the second stent body 50a comprises a second stent body and a third covering membrane provided on the outer wall of the second stent body, that is, the third covering membrane is sutured to the outer wall of the second stent body. Furthermore, the diameter of the second stent body 50a is equal to or slightly larger than the inner diameter of the first stent body 20a, such that the second stent body 50a and the first stent body 20 are firmly attached without endoleak.

In some embodiments, the second stent body (50a, 50b) has a flexible radial direction, which can be obtained, for example, by grinding the outer surface of the second stent body (50a, 50b). The proximal side of the second stent body (50a, 50b) is provided with first barbs 51a (correspondingly, first barbs 51b on the second stent body 50b), such that the second stent body 50a (or the second stent body 50b) is fastened on the first stent body 20a (or the first stent body 20b). The distal side of the second stent body (50a, 50b) may be selectively provided with second barbs 52a (correspondingly, second barbs 52b on the second stent body 50b), such that the second stent body 50a (or the second stent body 50b) is better fastened on the first stent body 20a (or the first stent body 20b). In this way, the second stent body (50a, 50b) and the first stent body (20a, 20b) are sufficiently attached without displacement. This can better seal the bare stent segment 23a part of the first stent body 20a and isolate lesions of the aortic arch. After the second stent body 50a is implanted, the stent graft of the second stent body 50a may also be appropriately expanded by using a compliant balloon to ensure the sealing performance thereof.

Further, an end of the second stent body is provided with a second positioning stent, and the second stent body is connected to an end of the leakproof channel by means of the second positioning stent.

In some embodiments, the proximal side of the second stent body 50a may also be provided with a second positioning stent 53a, and the tips of the second positioning stent 53a converges from the proximal side to the distal side, so as to prevent the second positioning stent 53a from damaging the aorta wall, and facilitate the release of the second stent body 50a.

In some embodiments, the first leakproof channel 71a and the second leakproof channel 72a are correspondingly connected inside the second opening 215a and the second opening 223a, and the front end and the rear end of the second stent body 50a are detachably nested inside the first leakproof channel 71a and the second leakproof channel 72a, respectively. The proximal side of the first leakproof channel 71a is connected to the second opening 215a; and meanwhile, in the proximal-side stent graft segment 21a, an outer wall of a first part of the first leakproof channel 71a abuts against an inner wall of the proximal-side stent graft segment 21a; and an outer wall of a second part of the first leakproof channel 71a is attached to an outer wall of the first embedded stent graft 211a and an outer wall of the second embedded stent graft 212a. **The** distal side of the first leakproof channel 71a is connected to the second opening 223a; and meanwhile, in the distal-side stent graft segment 22a, an outer wall of a first part of the second leakproof channel 72a abuts against an inner wall of the distal-side stent graft segment 22a; and an outer wall of a second part of the second leakproof channel 72a is attached to an outer wall of the third embedded stent graft 221a.

In some embodiments, the first leakproof channel and the second leakproof channel may be formed by covering membranes.

When the S-C branch-first aortic and branch artery reconstruction system provided in the embodiments of the present disclosure is in use, the first stent body 20a and the second stent body 50a are respectively implanted into the aortic arch. The first stent body 20a is implanted into the aortic arch first while ensuring an open blood supply to the cerebral circulation, the reconstruction of blood vessels of branch arteries of the aortic arch is preferentially completed, such that the blood supply of the three branch arteries in the aortic arch segment is completely reconstructed, and then the second stent body 50a is placed into the first stent body 20a, which can ensure the construction of the aortic channel while isolating blood flow from lesion sites, thereby realizing the patency and sealing of the aorta and various branch arteries, no leakage of blood (to the lesion sites), and isolating lesions at the aortic arch. By means of the cooperation between the embedded channel of the first stent body 20a and the second stent body 50a, the proximal side and the distal side can achieve seamless sealing cooperation, thereby avoiding endoleak. By maintaining blood circulation during reconstruction of branches on the arch, ischemia is avoided, normal supply of cerebral blood flow during surgery is ensured, and cerebral ischemia is avoided, thereby reducing the incidence rate of cerebral infarction. After the branches are reconstructed, the second stent body 50a is used for jointly occluding and isolating lesions with respect to a proximal-side ascending aortic segment, a middle arcuate segment and a distal-side descending aortic segment; the operation is simple, and modular and complex connection steps are avoided, which objectively avoids linking endoleak between modules caused by operation difficulty and operation imprecision.

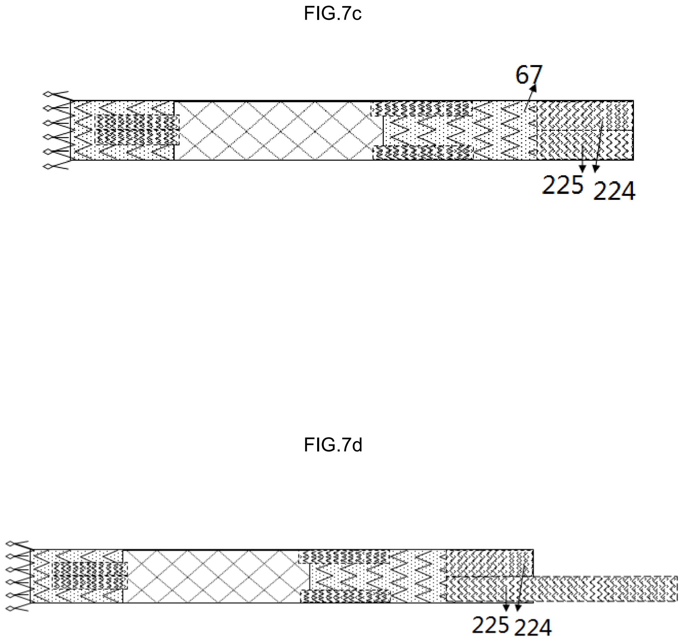

In some embodiments, the S-C branch-first aortic reconstruction system may be an S-C branch-first thoracoabdominal aortic reconstruction system, which has a structure similar to the S-C branch-first aortic arch reconstruction system. The aorta may comprise a thoracoabdominal aorta; and the branch arteries may be four branch arteries of thoracoabdominal viscera, comprising a celiac trunk artery branch, a superior mesenteric artery branch, a left renal artery branch, and a right renal artery branch. The branch artery stent grafts comprise a celiac trunk artery branch artery stent graft 61, a superior mesenteric artery branch artery stent graft 62, a left renal artery branch artery stent graft 63, and a right renal artery branch artery stent graft 64. The S-C branch-first thoracoabdominal aortic reconstruction system comprises a first stent body 20b, occlusion membranes and a second stent body 50b. As shown in Fig. 7-Fig. 8f, they are schematic diagrams of the first stent body 20b and the second stent body 50b being used in separation and in combination, respectively. The first stent body 20b comprises a bare stent segment 23b, and a proximal-side stent graft segment 21b and a distal-side stent graft segment 22b connected by the bare stent segment 23b. After being implanted into the body, the first stent body 20b crosses two ends of the arota involving lesions in visceral artery branch regions. The second stent body 50b is a stent graft body, and is configured to be nested in the bare stent segment 23b when the first stent body 20b is provided in the visceral branch regions of the thoracoabdominal aorta, such that the S-C branch-first aortic reconstruction system isolates lesion sites at the visceral artery regions.

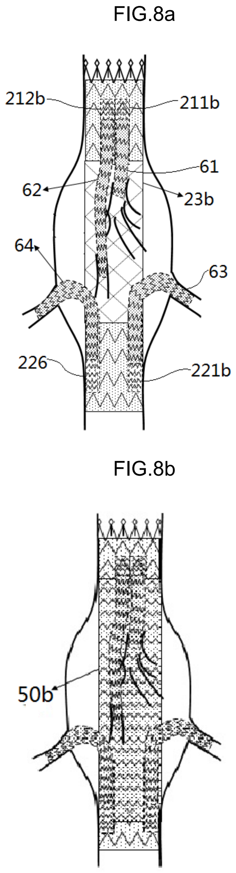

As shown in Fig. 8b, the relative positions of the celiac trunk artery branch artery stent graft 61, the superior mesenteric artery branch artery stent graft 62, the left renal artery branch artery stent graft 63, and the right renal artery branch artery stent graft 64 within the visceral artery branch regions substantially coincide with the positions of openings, in thoracoabdominal artery branch regions, of the celiac trunk artery branch, the superior mesenteric artery branch, the left renal artery branch, and the right renal artery branch in brach arteries on the thoracoabdominal artery branch regions, respectively.

In some embodiments, the proximal-side stent graft segment 21b may be internally provided with one embedded stent graft, and the distal-side stent graft segment 22b may be internally provided with three embedded stent grafts. In this way, a superior mesenteric artery branch channel can be constructed by blood reflowing in the embedded stent grafts in the distal-side stent graft segment 22b.

In some embodiments, two embedded stent grafts may be provided in each of the proximal-side stent graft segment 21b and the distal-side stent graft segment 22b. As shown in Fig. 7a, a first embedded stent graft 211b and a second embedded stent graft 212b in the proximal-side stent graft segment 21b are adjacent to each other, and are located on one side of the first leakproof channel 71b. In addition, a third embedded stent graft 221b and a fourth embedded stent graft 226 of the distal-side stent graft segment 22b are respectively attached to and arranged at two opposite sides of the second leakproof channel 72b in the distal-side stent graft segment 22b.

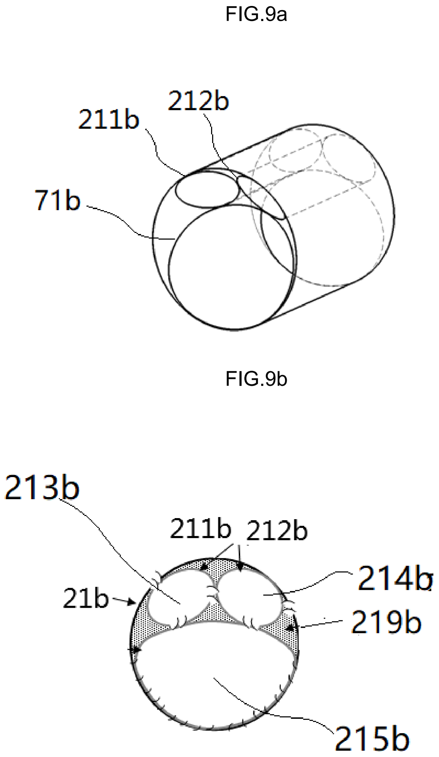

In some embodiments, the proximal-side stent graft segment 21b is internally provided with a proximal-side occlusion membrane 219b in a radial direction, the proximal-side occlusion membrane 219b is attached to an inner wall of the proximal-side stent graft segment 21b, and the proximal-side occlusion membrane 219b is provided with a first opening 213b, a first opening 214b and a second opening 215b to form a plurality of blood flow channels, as shown in Fig. 9a. The first opening 213b is sutured to a fourth embedded stent graft 211b, the fourth embedded stent graft 211b can pass through the bare stent segment 23b, and is nested with the celiac trunk artery branch artery stent graft 61 to form a reconstructed celiac trunk artery access. The first opening 214b is sutured to a fifth embedded stent graft 212b, and the fifth embedded stent graft 212b and the superior mesenteric artery branch artery stent graft 62 are nested to form a blood flow channel of a reconstructed superior mesenteric artery branch vessel. The second opening 215b is connected to the first leakproof channel 71b, the first leakproof channel 71b extends to the bare stent segment 23b, and blood can flow from the second opening 215b to the bare stent segment 23b.

In some embodiments, the distal-side stent graft segment 22b is internally provided with a distal-side occlusion membrane 229b in a radial direction, the distal-side occlusion membrane 229b is attached to an inner wall of the distal-side stent graft segment 22b, and the distal-side occlusion membrane 229b is internally provided with a first opening 222b, a first opening 25 and a second opening 223b, as shown in Fig. 9b. Accordingly, the first opening 222b is sutured to a sixth embedded stent graft 221b, the blood passes through the first opening 222b, flows into the sixth embedded stent graft 221b and then flows into a left renal artery branch vessel through the left renal artery branch artery stent graft 63 nested with the sixth embedded stent graft 221b; and the first opening 25 is connected to a seventh embedded stent graft 226, and the seventh embedded stent graft 226 is sleeved on an outer wall of the right renal artery branch artery stent graft 64, to form a reconstructed blood flow channel of a right renal artery branch vessel. In some embodiments, the second opening 223b is connected to the second leakproof channel 72b, the second leakproof channel 72b extends to the bare stent segment 23b, and blood can flow from the second opening 223b to the bare stent segment 23b. The second stent body 50b is nested within a first leakproof membrane and a second leakproof membrane, that is, two ends of the second stent body 50b are detachably connected to the first leakproof membrane and the second leakproof membrane, so as to achieve communication between the proximal side and the distal side of the first stent body 20b.

In this way, in an ischemia-free state, the reconstruction of a blood flow channel of the celiac trunk artery branch, the reconstruction of a blood flow channel of the superior mesenteric artery branch, and the reconstruction of blood flow channels of double renal artery branches can be realized, thereby completing the reconstruction of blood supply of the four branch arteries of thoracoabdominal viscera.

In some embodiments, in the proximal-side stent graft segment 21b, a part of an outer wall of the first leakproof membrane is attached to an inner wall of the proximal-side stent graft segment 21b; and the other part of the outer wall of the first leakproof membrane is attached to the outer walls of the fifth embedded stent graft 212b and the fourth embedded stent graft 211b. In the distal-side stent graft segment 22b, a part of an outer wall of the second leakproof membrane is attached to an inner wall of the distal-side stent graft segment 22b; and a part of the outer wall of the second leakproof membrane is attached to the outer walls of the sixth embedded stent graft 221b and the seventh embedded stent graft 226.

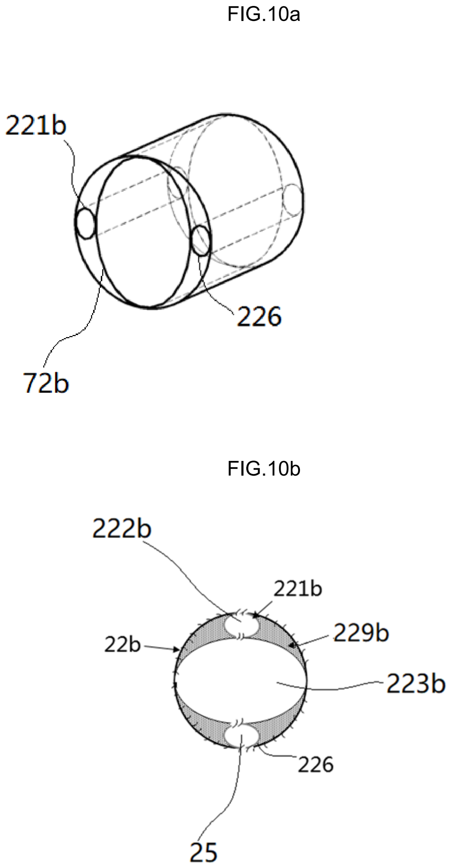

In some embodiments, as shown in Figs. 10a and 10b, the sixth embedded stent graft 221b and the seventh embedded stent graft 226 are distributed at positions on two symmetrical sides of the distal-side stent graft segment 22b. That is, the sixth embedded stent graft 221b and the seventh embedded stent graft 226 are respectively attached to two opposite sides of the distal-side stent graft segment 22b in the radial direction. Each of the sixth embedded stent graft 221b and the seventh embedded stent graft 226 may be independently circular or elliptical. The sixth embedded stent graft 221b and the seventh embedded stent graft 226 may respectively slightly protrude beyond the proximal-side stent graft segment 21b and enters the region of the bare stent segment 23b (e.g. the length entering the region of the bare stent segment 23b may be about 5 mm), so as to be better nested with the left renal artery branch artery stent graft 63 and the right renal artery branch artery stent graft 64. Of course, it is only taken as an example, and it does not mean that only the sixth embedded stent graft 221b and the seventh embedded stent graft 226 can slightly protrude beyond the proximal-side stent graft segment 21b.

Specifically, the material of the covering membrane in each of the stent grafts may be polytetrafluoroethylene, etc. However, the materials of the occlusion membranes and the leakproof channels may be the same as those of the covering membranes. Each covering membrane may be sutured to a stent. Each of the leakproof channels may have the same shape or different shapes. For example, each of the leakproof channels may be an annular leakproof membrane, and sutured in a sleeve type on the surface of an adjacent covering membrane, and the surface between a covering membrane and an adjacent stent.

Further, the distal end of the distal-side stent graft segment is provided with an extension section, the extension section being internally provided with two second stent graft channels provided in parallel; one part of each of the second stent graft channels is provided in the extension section, and the other part thereof extends out of the extension section and is in communication with a corresponding branch artery stent graft, and the two second stent graft channels fill the extension section. In some embodiments, the cross sections of the two second stent graft channels form a double D-shape.

In some embodiments, as shown in Figs. 7c and 7d, a distal-side stent graft extension section 67 is further provided at the distal side of the first stent body 20b, and the distal end of the distal-side stent graft extension section 67 is connected to second stent graft channels (224, 225); and the second stent graft channels (224, 225) are in communication with an external iliac artery branch artery stent graft 65 and an internal iliac artery branch artery stent graft 66, respectively. The second stent graft channels (224, 225) are respectively attached to and provided in the distal-side stent graft segment extension section 67 and is adjacent and parallel to each other, and the distal side of the second stent graft channel 224 is coaxially nested with the proximal side of the external iliac artery branch artery stent graft 65; and the distal side of the second stent graft channel 225 is coaxially nested with the proximal side of the external iliac artery branch artery stent graft 65. In this way, reconstruction for situations in which aortic lesions extend to the aorta and branch arteries in an abdominal aortic region can be achieved.

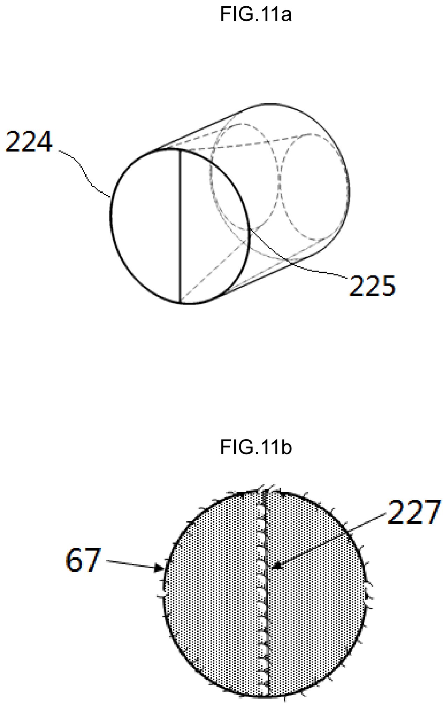

In some embodiments, as shown in Fig. 11a and Fig. 11b, adjacent side walls of the proximal side of the second stent graft channel 224 and the proximal side of the second stent graft channel 225 are attached to each other; and non-adjacent side walls of the proximal side of the second stent graft channel 224 and the proximal side of the second stent graft channel 225 are attached to the inner wall of the second stent graft segment extension section 67. It can be understood that, the proximal side of the second stent graft channel 224 and the proximal side of the second stent graft channel 225 (in the direction of the blood flow) are end-to-end sutured together to form a double-"D" shape, so as to ensure leakage-free sealing, thereby gradually and naturally transitioning to an elliptical or circular shape of the distal end. In this way, the second stent graft channel 224 and the second stent graft channel 225 and the distal-side stent segment extension section are sutured by covering membranes without slits, to ensure that no leakage of blood occurs.

In some embodiments, in the radial direction, the size of the distal side of the second stent graft channel 224 is the same as the size of the proximal side of a branch artery stent graft of the external iliac artery branch artery stent graft 65; and the size of the distal side of the second stent graft channel 225 is the same as the size of the proximal side of the internal iliac artery branch artery stent graft 66.

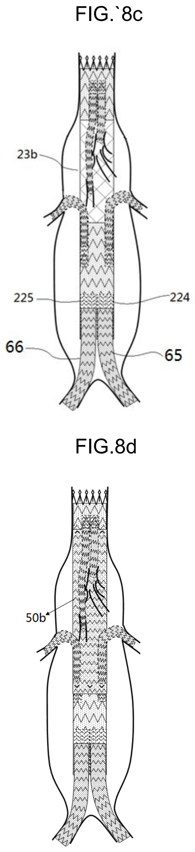

In some embodiments, as shown in Fig. 8c and Fig. 8d, the proximal side of the external iliac artery branch artery stent graft 65 is nested in the distal side of the second stent graft channel 224; and the proximal side of the internal iliac artery branch artery stent graft 66 is nested in the distal side of the second stent graft channel 225.

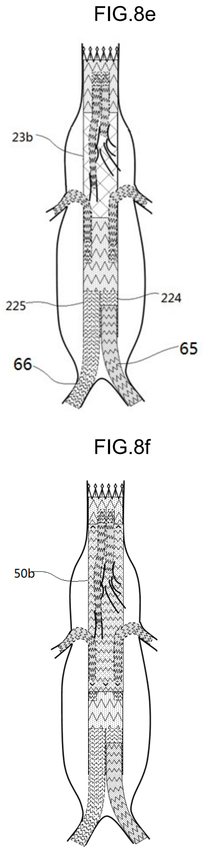

In some embodiments, as shown in Fig. 7d, in the axial direction, the size of the second stent graft channel 224 is less than the size of the second stent graft channel 225. In this way, as shown in Fig. 8e and Fig. 8f, the proximal side of the external iliac artery branch artery stent graft 65 is nested in the distal side of the second stent graft channel 224; and the proximal side of the internal iliac artery branch artery stent graft 66 is nested in the distal side of the second stent graft channel 225.

Embodiments of the present disclosure further provide an S-C branch-first aortic reconstruction system, comprising the branch artery reconstruction assist device in any one of the embodiments and the second stent body; wherein the second stent body is a stent graft for forming an aortic channel, and is used for being provided within a first stent body after completing positioning and releasing of the first stent body and completing the reconstruction of branch arteries, to form the aortic channel, so as to communicate with blood flow within the aorta while isolating lesion sites.

In the S-C branch-first aortic reconstruction system, two ends of the second stent body are respectively in communication with the second opening on the occlusion membrane in the proximal-side stent graft segment and the second opening on the occlusion membrane in the distal-side stent graft segment.

The S-C branch-first thoracoabdominal aortic reconstruction system provided in the embodiments of the present disclosure, the system is implanted separately in two parts, in which the first stent body preferentially completes the reconstruction of branch vessels while ensuring an open blood supply to circulation of visceral organs; and the second stent body cooperates with the first stent body to complete the construction of the aortic channel and isolate lesions at the region of the branch arteries. The first stent body is divided into three parts integrated as a whole. The proximal-side stent graft segment 21b is a stent graft and is internally embedded with two adjacent stent grafts (i.e. the first embedded stent graft 211b and the second embedded stent graft 212b). The tips of the second positioning stent 53a of the proximal-side stent graft segment 21b are designed to be an inwardly-retracted shape so as to prevent the tips from puncturing an ascending aortic wall; furthermore, barbs are sutured to an edge of a covering membrane of the front end of the proximal-side stent graft segment 21b, to prevent displacement. Two symmetrical stent grafts (i.e. the third embedded stent graft 221b and the fourth embedded stent graft 226) are embedded in the distal-side stent graft segment 22b, and a middle connecting segment (i.e. the bare stent segment 23b) is a sparse-mesh bare stent. When embedded branch channels of the stent graft segments (comprising the proximal-side stent graft segment 21b and the distal-side stent graft segment 22b) of the first stent body are sutured with a main stent graft, it is ensured that sealing is achieved without gaps; and an inward-facing segment of the first stent body and the main stent graft keep regular elliptical shapes, so as to form a seamless fit with the stent graft of the implanted second stent body, thereby achieving the purpose of proximal-end sealing. The second stent body is used for sealing and isolating lesions, is radially flexible, and is provided with barbs preventing displacement at the proximal and distal ends, so as to ensure that the second stent body is seamlessly attached to and firmly bonded with the stent graft of the first stent body having an embedded channel.

When the S-C branch-first thoracoabdominal aortic reconstruction system provided in the embodiments of the present disclosure is in use, in principle, the first stent body 20b and the second stent body 50b are implanted successively into the visceral branch regions of the thoracoabdominal aorta having lesions. The first stent body 20b is implanted into visceral arteries in the aorta first under normal conditions of ensuring open blood supply to visceral organs, and then various branch artery stent grafts are implanted respectively, so as to preferentially complete the reconstruction of vessels of branch arteries of the visceral arteries in the aorta; and then, the second stent body 50b is implanted into the first stent body 20b in a cooperative manner, and covers the first stent body 20b in an overlapping manner, thereby isolating lesions of the thoracoabdominal aorta. By means of the cooperation between the first stent body 20b and the second stent body 50b, the proximal side and the distal side can achieve seamless sealing cooperation, thereby avoiding endoleak. During reconstruction of the aorta and visceral artery branches, the entrances of the branch arteries of the visceral arteries are all located at parts of the bare stent segment 23b where no covering membrane is provided, and in an ischemia-free state, the branch arteries are respectively linked to the celiac trunk artery, the superior mesenteric artery, and the left and right renal arteries by means of embedded stent grafts, and branch stent grafts are implanted; after reconstruction of blood supply of the four branch arteries of the thoracoabdominal viscera is completed, the blood circulation is maintained, ischemia is avoided, normal supply of cerebral blood flow during surgery is ensured, , cerebral hemorrhage is avoided and thus reduce the incidence of cerebral infarction; and then a soft second stent body 50b is placed to overlap with the first stent body 20b, so as to seal lesions at the visceral artery regions; moreover, the patency of various branch is ensured, thereby reducing the incidence rate of complications and the like. After the branches are reconstructed, the second stent body 50b is used for jointly occluding and isolating lesions with respect to a proximal-side ascending aortic segment, a middle arcuate segment and a distal-side descending aortic segment; the operation is simple, and modular and complex connection steps are avoided, which objectively avoids linking endoleak between modules caused by operation difficulty and operation imprecision.

It should be understood by a person of ordinary skill in the art: the discussion of any embodiment above is merely exemplary, and is not intended to imply that the scope (comprising the claims) disclosed in the present disclosure is limited to these examples. Under the idea of the present disclosure, technical features in the described embodiments or in different embodiments may also be combined, the steps can be implemented in any sequence; and there are many other variations of different aspects of the embodiments of the present disclosure as described above, but for simplicity, they are not provided in detail.

Although the present disclosure has been described in conjunction with specific embodiments of the present disclosure, many replacements, modifications and variations of these embodiments would be apparent to a person of ordinary skill in the art in light of the illustration above.

The embodiments of the present disclosure are intended to cover all such replacements, modifications and variations falling within the broad scope of the appended claims. Therefore, any omissions, modifications, equivalent replacements, improvements, etc. made within the spirit and principle of embodiments of the present disclosure shall all fall within the scope of protection of the present disclosure.

## Claims

1. A branch artery reconstruction assist device, comprising: a first stent body; the first stent body comprises a bare stent segment, and a proximal-side stent graft segment and a distal-side stent graft segment connected to two ends of the bare stent segment; wherein each of the proximal-side stent graft segment and the distal-side stent graft segment is provided with an occlusion membrane and at least one first stent graft channel; the occlusion membrane comprises a first opening, and the first opening is correspondingly connected to a first end of the first stent graft channel, and a second end of the first stent graft channel is connected to a corresponding branch artery stent graft to form a branch channel so as to pass through the bare stent.

2. The branch artery reconstruction assist device according to claim 1, wherein the distance between an inlet end of a branch artery corresponding to the proximal-side stent graft segment and an inlet end of a branch artery corresponding to the distal-side stent graft segment is smaller than the length of the bare stent.

3. The branch artery reconstruction assist device according to claim 1, wherein the bare stent has a bendable structure.

4. The branch artery reconstruction assist device according to claim 1, wherein the connection position between the proximal-side stent graft segment or the distal-side stent graft segment and the occlusion membrane is flush with the first end of the first stent graft channel, the occlusion membrane is in a planar shape; and/or when the connection position between the proximal-side stent graft segment or the distal-side stent graft segment and the occlusion membrane is not flush with the first end of the first stent graft channel, the occlusion membrane is in an inclined surface shape.

5. The branch artery reconstruction assist device according to claim 4, wherein the occlusion membrane in the proximal-side stent graft segment is connected to a proximal end of the proximal-side stent graft segment, and the occlusion membrane in the distal-side stent graft segment is connected to a distal end of the distal-side stent graft segment.

6. The branch artery reconstruction assist device according to claim 1, wherein a first positioning stent is provided at the proximal end of the proximal-side stent graft segment and/or at the distal end of the distal-side stent graft segment.

7. The branch artery reconstruction assist device according to any one of claims 1-6, wherein the occlusion membrane further comprises a second opening, and the second opening of the occlusion membrane in the proximal-side stent graft segment and the second opening of the occlusion membrane in the distal-side stent graft segment are in communication with each other through an aortic channel.

8. The branch artery reconstruction assist device according to claim 7, wherein the first opening is provided on one side of the occlusion membrane, and the second opening is provided on the other side of the occlusion membrane.

9. The branch artery reconstruction assist device according to claim 7, wherein the second opening is provided in the middle position of the occlusion membrane, and the first openings are provided on both sides of the second opening.

10. The branch artery reconstruction assist device according to claim 7, wherein the distal end of the distal-side stent graft segment is provided with an extension section, the extension section being internally provided with two second stent graft channels provided in parallel; one part of each of the second stent graft channels is provided in the extension section, and the other part thereof extends out of the extension section and is in communication with a corresponding branch artery stent graft, and the two second stent graft channels fill the extension section.

11. The branch artery reconstruction assist device according to claim 10, wherein the cross sections of the two second stent graft channels form a double D-shape.

12. An S-C branch-first aortic reconstruction system, comprising the branch artery reconstruction assist device according to any one of claims 7-11 and a second stent body, wherein the second stent body is a stent graft for forming an aortic channel, and is used for being provided within a first stent body after completing positioning and releasing of the first stent body and completing the reconstruction of branch arteries, to form the aortic channel, so as to communicate with blood flow within the aorta while isolating lesion sites.

13. The S-C branch-first aortic reconstruction system according to claim 12, wherein two ends of the second stent body are respectively in communication with the second opening on the occlusion membrane in the proximal-side stent graft segment and the second opening on the occlusion membrane in the distal-side stent graft segment.

14. The S-C branch-first aortic reconstruction system according to claim 12, wherein a leakproof channel is provided in the proximal-side stent graft segment and/or the distal-side stent graft segment, wherein one end of the leakproof channel is connected to the second opening, and the other end of the leakproof channel is connected to an end corresponding to the second stent body.

15. The S-C branch-first aortic reconstruction system according to claim 14, wherein an end of the second stent body is provided with a second positioning stent, and the second stent body is connected to an end of the leakproof channel by means of the second positioning stent.
